# EUROPÄISCHE PATENTSCHRIFT

(11) **EP 1 669 018 B1**
(45) Veröffentlichungstag und Bekanntmachung des Hinweises auf die Patenterteilung: **04.03.2009**
(21) Anmeldenummer: 05026722.8
(22) Anmeldetag: 07.12.2005
(51) Int. Cl.: A61B 1/005, A61M 25/01

(54) **Endoskop mit rotierbarem distalen Endoskopkopf**
Endoscope with rotatable distal endoscope head
Endoscope avec une tête d'endoscope rotative

(30) Priorität: 07.12.2004 DE 102004058929
(43) Veröffentlichungstag der Anmeldung: 14.06.2006
(73) Patentinhaber: Invendo Medical GmbH, 69469 Weinheim (DE)
(72) Erfinder: Bob, Konstantin, Dr., 69469 Weinheim (DE); Pauker, Fritz, 86438 Kissing (DE); Viebach, Thomas, 82282 Pischertshofen (DE)
(74) Vertreter: TBK-Patent

(56) Entgegenhaltungen:
- FR-A- 2 838 325
- US-A- 4 976 191
- US-A- 5 019 121
- US-A1- 2003 181 785

## Beschreibung

Die vorliegende Erfindung betrifft ein Endoskop mit rotierbarem distalem Endstück gemäß dem Oberbegriff des Patentanspruchs 1.

Aus dem Stand der Technik sind Endoskope der vorliegenden Erfinderin selbst bekannt, deren jeweiliges distales Endstück in Form eines sog. Deflecting unter einem engen Biegeradius zu allen Seiten hin abwinkelbar ist. Das Deflecting dient im wesentlichen dazu, den Einfahrvorgang des Endoskops bspw. in den Darm eines Patienten zu erleichtern, indem das Deflecting entsprechend den einzelnen Biegungen des Darms dynamisch angepasst werden kann und somit der Einschiebvorgang erheblich erleichtert und damit für den Patienten weniger schmerzhaft wird.

An der Endoskopspitze befindet sich für gewöhnlich ein sog. Endoskopkopf, in welchem eine Mehrzahl von für eine Endoskopie erforderlichen Einrichtungen angeordnet sind. Hierzu zählen unter anderem eine Beleuchtungs- sowie eine Spülvorrichtung und natürlich ein lichtempfindliches Element, bspw. in Form einer Optik, eines lichtempfindlichen elektronischen Bauteils oder eines Lichtleiters. Die Optik dient dabei dazu, den Blickwinkel zu erweitern, um einen möglichst großen Bereich der den Endoskopkopf umgebenden Darmwand betrachten zu können.

Aus der vorstehenden kurzen Beschreibung dieses komplexen Bauteils wird ersichtlich, dass eine Vielzahl technischer Vorrichtungen insbesondere am distalen Endbereich des Endoskops angeordnet sein müssen, um dessen Funktionsfähigkeit zu gewährleisten. Demgegenüber steht jedoch ein nur verhältnismäßig kleiner zur Verfügung stehender Bauraum, der zu einer Miniaturisierung sämtlicher Bauteile zwingt. Dieser Miniaturisierungen sind jedoch zwangsläufig Grenzen gesetzt, da ab einem bestimmten Miniaturisierungsgrad die Funktionsfähigkeit insbesondere der mechanischen Bauteile nicht mehr gewährleistet werden kann.

Aus der FR 2 838 325 ist ein Endoskop mit einem Endoskopkopf und einem abwinkelbaren distalen Endstück bekannt, an dem der Endoskopkopf montiert ist. Das abwinkelbare distale Endstück ist zusammen mit dem Endoskopkopf mittels eines Rotationselements um die Längsachse der Endoskops drehbar.

Ferner offenbart auch die US 5 019 121 A einen helixförmig aufgebauten hydraulischen Aktuator, welcher bei Beaufschlagung von Druck eine rotatorische Bewegung ausführt.

Angesichts dieser technischen Situation besteht die Notwendigkeit, ein Endoskop zu schaffen, welches die vorstehend genannten Eigenschaften moderner Endoskope erfüllt, bei dem jedoch ein geringerer Bauraum erforderlich ist bzw. der zur Verfügung stehende Bauraum besser genutzt wird.

Diese Aufgabe wird erfindungsgemäß durch ein Endoskop mit den Merkmalen des Patentanspruchs 1 gelöst. Weitere vorteilhafte Ausgestaltungen der Erfindung sind dabei Gegenstand der übrigen Unteransprüche.

Das Grundprinzip der Erfindung besteht demzufolge darin, den Bewegungsapparat des vorstehend definierten Deflecting in einen Rotations- und einen Abkrümmmechanismus aufzugliedern und beide Mechanismen im Axialabstand zueinander anzuordnen. Durch dieses Erfindungsprinzip kann jener Bauraum, der in Endoskoplängsrichtung reichlich zur Verfügung steht, besser ausgenutzt werden, wohingegen jener Ba-uraum, der in einem Axialabschnitt des Endoskops in Radialrichtung nur eingeschränkt vorhanden ist, nunmehr nicht mehr gänzlich ausgenutzt werden muss, um alle notwendigen Einrichtungen des Endoskops unterzubekommen. Der wesentliche Vorteil dieses Erfindungsprinzips besteht demzufolge darin, dass durch eine hierdurch ermöglichte Reduktion der äußeren radialen Abmessungen insbesondere im Bereich des distalen Endabschnitts des Endoskops noch kleinere Biegeradien erreichbar sind, und damit der Einsatzbereich des Endoskops erweitert wird.

Konkreter ausgedrückt hat das Deflecting des erfindungsgemäßen Endoskops einen Abkrümmmechanismus vorzugsweise in Form eines solchen (einzelnen) Faltenbalgs, der eine Abkrümmbewegung in im wesentlichen (vorzugsweise) nur eine einzige Richtung bzw. in nur einer Krümmungsebene (zweidimensional) ermöglicht. Da eine Abkrümmbewegung nach allen Richtungen nur durch eine Mehrzahl von derartigen Abkrümmmechanismen vorzugsweise in Form von Faltenbälgen möglich ist, kann also erfindungsgemäß auf eine Anzahl von Abkrümmmechanismen verzichtet werden. Statt dessen ist ein Rotationselement axialversetzt (unterhalb) zu dem Deflecting angeordnet, mittels dem das Deflecting um dessen Längsachse oder die Längsachse des Endoskopschafts rotiert werden kann. Auf diese Weise kann durch die überlagerten Bewegungen des Abkrümm- und Rotationsmechanismus das Deflecting folglich nach allen Richtungen abgekrümmt werden.

Erfindungsgemäß ist das Rotationselement ein Koaxialantrieb nämlich eine Turbine, sodass ein Arbeitskanal des Endoskops, welches in der Regel mittig im Endoskopschaft verläuft, auch durch das Rotationselement geführt werden kann. Darüber hinaus kann durch einen solchen Antrieb ein ausreichendes Drehmoment erzeugt werden, um sich gegen einen mechanischen Widerstand innerhalb einer Körperhöhlung ausrichten zu können. Schließlich können diese Antriebe für ein Einweggerät kostengünstig hergestellt werden.

Um die relativ hohen Drehzahlen einer solchen Turbine für eine möglichst exakt steuerbare Rotation des Deflecting besser nutzbar zu machen, kann ein Untersetzungsgetriebe, vorzugsweise ebenfalls in Axialbauweise angeordnet sein. Hierfür kommen im wesentlichen Getriebe mit hoher Untersetzung (selbsthemmend) wie das sogenannte Harmonic drive, Planetenradsätze oder Cykloidengetriebe zur Anwendung. Diese Getriebe haben den wesentlichen Vorteil der koaxialen Anordnungsmöglichkeit bezüglich der Turbine, wodurch quasi zwangsläufig zentrisch verlaufende Durchgangsöffnungen im Getriebe geschaffen werden, durch welche die Arbeits- und Versorgungskanäle für den Endoskopkopf hindurch geführt werden können.

Die vorstehend genannten Komponenten können darüber hinaus sämtlich vorteilhaft in kostengünstiger Spritzgußtechnik hergestellt werden. Elektrische Leitungen für im Endoskopkopf befindliche Bauteile wie beispielsweise Karnerachips, LED's usw., welche ggf. nicht innerhalb der vorstehend genannten Durchgangsbohrung geführt sind, können über Schleifringe elektrisch gekoppelt werden.

Die Erfindung wird nachstehend anhand eines bevorzugten Ausführungsbeispiels unter Bezugnahme auf die begleitenden Zeichnungen näher erläutert.
Fig. 1 zeigt eine Perspektivenansicht des distalen Endstücks des erfindungsgemäßen Endoskops;
Fig. 2 zeigt einen prinzipiellen Seitenriß eines Teilbereichs des distalen Endstücks eines Endoskops gemäß der Erfindung;
Fig. 3 zeigt den generellen Grundriß eines sogenannten Harmoniedrive; und
Fig. 4 zeigt den schematischen Seitenriß eines Endoskopkopfs.

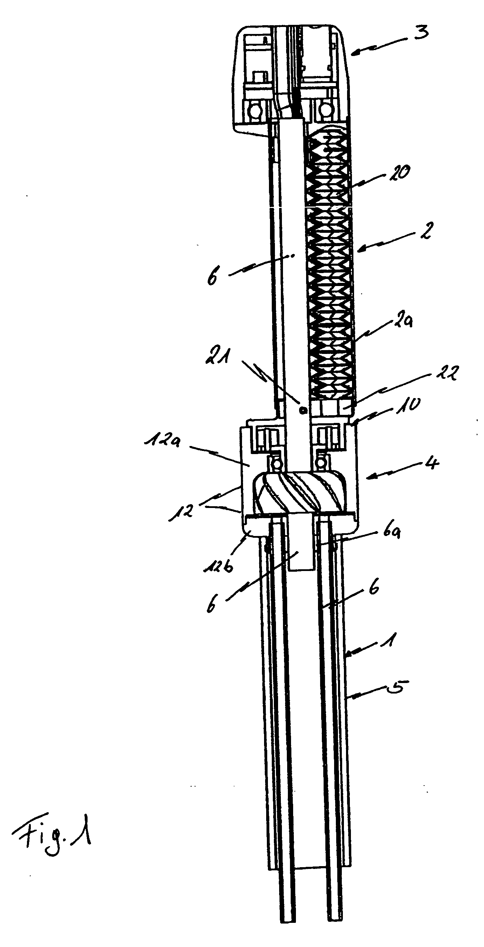

Gemäß der Fig. 1 besteht das Endoskop gemäß dem bevorzugten Ausführungsbeispiel dieser Erfindung aus einem Endoskopschaft 1, dessen distales Endstück aus einem abkrümmbaren Betätigungsteil (nachfolgend als "Deflecting" bezeichnet) 2, einem Endoskopkopf 3 sowie einem Rotationselement 4 gebildet wird. Das Rotationselement 4 befindet sich dabei zwischen dem Endoskopschaft 1 sowie dem Deflecting 2 und dient dazu, das Deflecting 2 sowie den axial daran sich anschließenden Endoskopkopf 3 rotatorisch um die Längsachse des Deflecting 2 bzw. des Endoskopschafts 1 zu bewegen.

Im vorliegenden Ausführungsbeispiel ist der Endoskopschaft 1 sowie das Rotationselement 4 als separate Bauteile ausgebildet, wobei diese zur Herstellung eines Endoskops bspw. über einen stirnseitigen Bayonett- oder Schraubverschluss montiert sind. Diese Montage könnte natürlich auch durch Verkleben, Verschweißen oder durch einen Steckmechanismus erfolgen, wie nachfolgend noch im Einzelnen beschrieben wird. Alternativ ist es natürlich auch möglich, das Rotationselement 4 quasi als Bestandteil des Endoskopschafts 1 in diesen zu integrieren.

Gemäß der Fig. 1 hat der Endoskopschaft 1 unter anderem eine vorzugsweise zylinderförmige oder schlauchartige Außenwand 5 sowie ein koaxial zu dieser verlaufendes inneres Rohr 6, wodurch sich ein im Querschnitt kreisringförmiger Bereich ausbildet, in welchem eine Mehrzahl von nicht gezeigten Arbeits- sowie Versorgungskanälen, Leitungen und dgl. angeordnet sind, welche nachfolgend nicht näher beschrieben werden. Der Aufbau des Endoskopschafts 1 entspricht somit im wesentlichen dem Schaftaufbau bereits bekannter Endoskope, wie sie auch Gegenstand von früheren Patentanmeldungen der Erfinderin selbst sind.

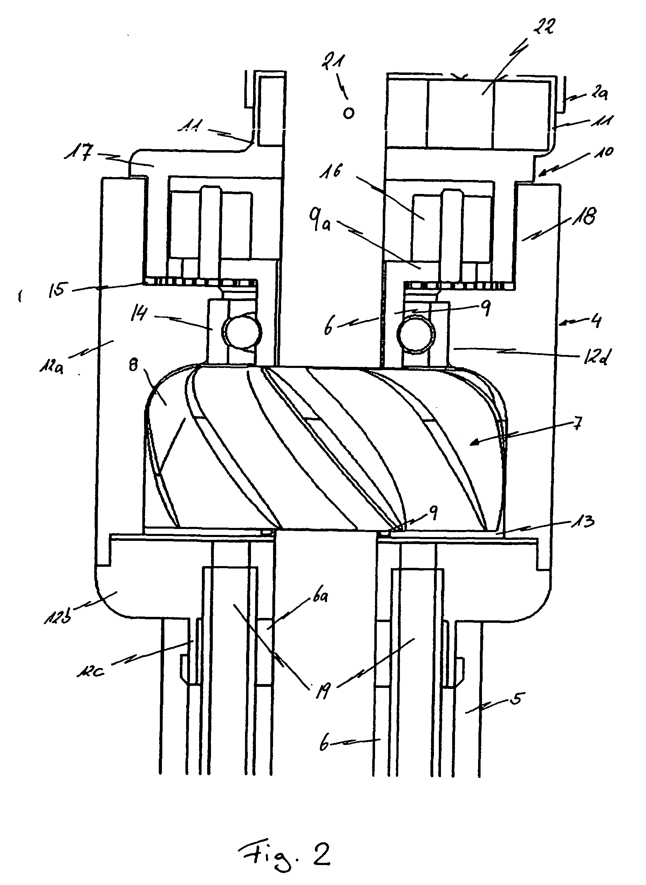

An der vorderen Stirnseite dieses Endoskopschafts 1 schließt sich, wie vorstehend bereits kurz angedeutet wurde, das Rotationselement 4 an. Vorliegend hat das Rotationselement 4 gemäß der Fig. 2 eine Turbine 7, deren Abtriebswelle 9 mit einem Untersetzungsgetriebe 10 verbunden ist, dessen Abtriebswelle 11 wiederum mit dem axial nachfolgenden Deflecting 2 rotationsfest verbunden ist.

Des weiteren hat das Rotationselement 4 ein Gehäuse 12, welches vorliegend einen äußeren zylindrischen Mittenabschnitt 12a aufweist, der an seiner dem Endoskopschaft 1 zugewandten Seite durch einen einen Anschlussstutzen 12c aufweisenden Deckel 12b verschlossen ist. Es sei dabei darauf hingewiesen, dass der Deckel 12b eine mittige Durchgangsbohrung aufweist, durch die das innere Rohr 6 des Endoskopschafts 1 geführt ist.

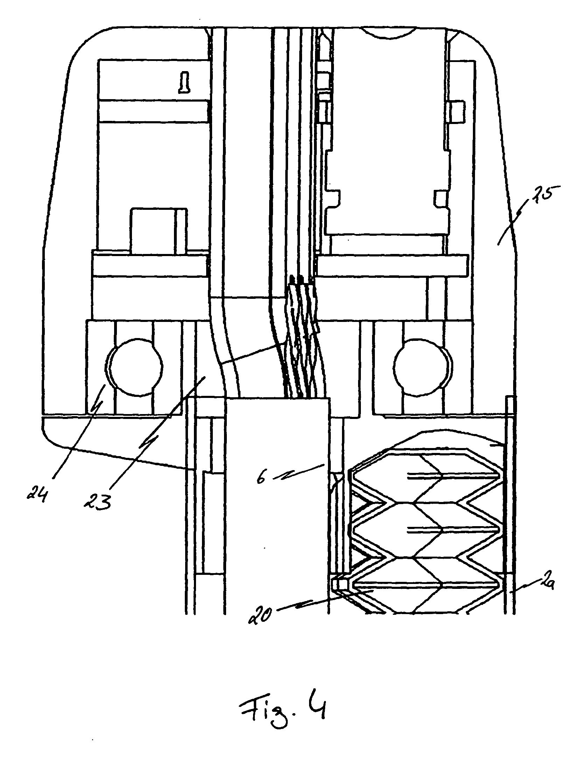

Wie insbesondere aus der Fig. 4 zu ersehen ist, bildet der Anschlussstutzen 12c einen axial in Richtung zum Endoskopschaft 1 sich erstreckenden Rohrstumpf, an dessen freiem Ende Rastelemente beispielsweise in Form von radial nach außen vorragenden Vorsprüngen ausgebildet sind. Dieser Rohrstumpf 12c ist mittels der Rastelemente fest im Endoskopschaft 1 verankert, indem der Rohrstumpf 12c stirnseitig in den Endoskopschaft 1 eingedrückt ist, wodurch sich die Rastelemente in dem Schaftmaterial verhaken. Der zylindrische Mittenabschnitt 12a des Gehäuses 12 bildet dabei zusammen mit dem Deckel 12b eine Aufnahmekammer 13 für die Turbine 7.

Im einzelnen wird die Turbine 7 aus einem Turbinenrad 8 sowie einem Stator gebildet, welcher vorliegend durch den Mittenabschnitt 12a des Gehäuses 12 gebildet wird. Das Turbinenrad 8 ist mit einer zentralen Bohrung versehen, deren Durchmesser so dimensioniert ist, dass ein Rohr, welches eine Verlängerung des im Endoskopschaft 1 verlaufenden inneren Rohrs 6 darstellt, frei (d.h. relativ drehbar zum Turbinenrad 8) hindurchverlaufen kann. Vorzugsweise ist dieses Verlängerungsrohr in das innere Rohr 6 des Endoskopschafts eingesteckt und mittels einer Ringdichtung 6a abgedichtet. Alternativ hierzu kann es sich bei diesem Verlängerungsrohr auch um das innere Rohr 6 des Endoskopschafts 1 selbst handeln.

Am Turbinenrad 8 ist ferner ein das innere Rohr 6 umgebender Rohrschaft angeordnet, der sich axial in Richtung zum Deflecting 2 erstreckt und auf dem ein Wälzlager 14 unmittelbar benachbart zum Turbinenrad 8 aufsitzt. Dieser Rohrschaft bildet dabei die Abtriebswelle 9 oder Rotor der Turbine 7. Das Wälzlager 14 stützt sich ferner an einem mittigen, radial nach innen gerichteten Vorsprung 12d des Mittenabschnitts 12a ab und hält so das Turbinenrad 8 drehbar am Gehäuse 12. Dieser mittige Vorsprung 12d begrenzt im übrigen die Aufnahmekammer 13 für die Turbine 7 in axialer Richtung und trennt diese gleichzeitig von einer axial nachfolgenden Getriebekammer 15.

Erfindungsgemäß ist das vorstehend genannte Untersetzungsgetriebe 10 in Form eines koaxialen Getriebes, insbesondere eines Planetengetriebes oder eines sog. "Harmonic drive" mit hoher Untersetzung und somit als selbsthemmendes Getriebe ausgeführt. Dieses Getriebe 10 ist in der Getriebekammer 15 untergebracht, welche ebenfalls vom Mittenabschnitt 12a des Gehäuses 12 des Rotationselements 4 umschlossen ist.

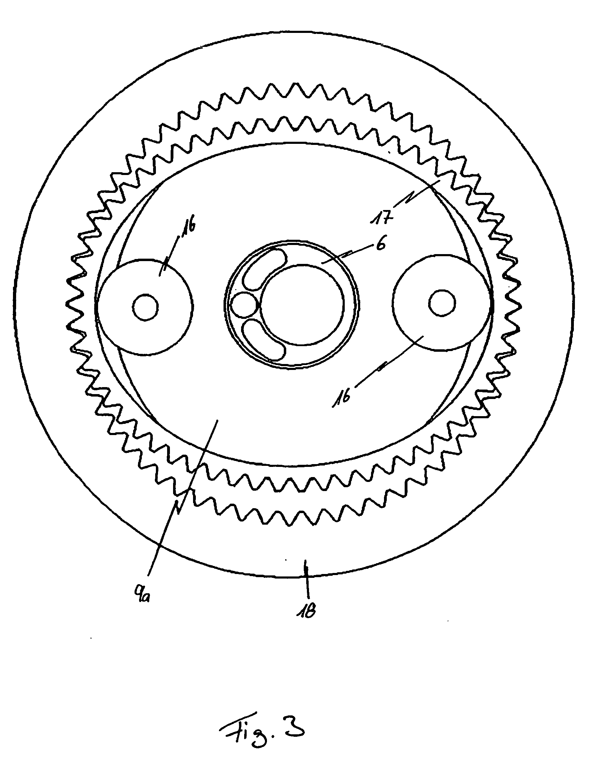

Im vorliegenden Ausführungsbeispiel gemäß der Fig. 2 bildet der mit der Turbine 7 fest verbundene Rohrschaft 9 demzufolge an seinem axial freien Ende einen Radträger 9a für die Andrückräder 16 des sogenannten Harmoniedrive 10. Das Grundprinzip dieses Harmoniedrive 10 ist aus dem Stand der Technik hinlänglich bekannt und soll daher nur allgemein anhand der anliegenden Fig. 3 beschrieben werden.

Gemäß der Fig. 3 besteht ein solcher Harmoniedrive 10 im wesentlichen aus dem vorstehend genannten Radträger 9a, an dem zumindest zwei Andrückräder 16 gelagert sind. Diese Andrückräder 16 sind exzentrisch bezüglich der Rotationsachse der Radträgers 9a angeordnet und sind mit der Innenseite eines Außenzahnrads 17 in Eingriff, welches sich dementsprechend ebenfalls exzentrisch bezüglich des Radträgers 9a ausrichtet. Das Außenzahnrad 17 steht wiederum mit einem fixen Hohlrad 18 in Kämmeingriff, derart, dass bei einer Rotation des Radträgers 9a eine Exzenterbewegung des Außenzahnrads 17 einsetzt, wodurch das Außenzahnrad 17 infolge des Zahneingriffs mit dem Hohlrad 18 in eine bezüglich der Rotation des Radträgers 9a stark untersetzte Rotationsbewegung gezwungen wird. Ein ähnliches Untersetzungsprinzip ist im übrigen auch bei sogenannten Cycloidengetrieben bekannt, so dass der vorstehend beschriebene Harmoniedrive 10 durch ein per se bekanntes Cycloidengetriebe ersetzt werden könnte.

Auch ist es möglich, ein einfaches Planetengetriebe vorzusehen. In einer solchen alternativen nicht weiter gezeigten Ausführungsform bildet demnach der vorstehend beschriebene Rohrschaft 9 an seinem freien Ende ein Sonnenrad, welches mit einer Anzahl von Planetenrädern in Wirkeingriff ist. Ein Planetenradträger ist in einem solchen Fall vorzugsweise mit einer Abtriebswelle des Getriebes fest verbunden, welche sich axial in Richtung zum Deflecting 2 erstreckt. Die Planetenräder sind wiederum mit einem Hohlrad in Wirkeingriff, welches vorzugsweise vom Mittenabschnitt 12a des Gehäuses 12 des Rotationselements 4 gebildet wird.

Wie aus der Fig. 1 ferner zu entnehmen ist, sind in dem Ringspalt zwischen Außenwand 5 und Innenrohr 6 im Bereich des Endoskopschafts 1 zumindest zwei vorzugsweise diametral gegenüberliegende Kanäle 19 angeordnet, welche am Übergangsbereich zwischen Endoskopschaft 1 und Rotationselement 4 in die Turbinen-Aufnahmekammer 13 münden. Dabei bildet der eine Kanal einen Einlass und der andere Kanal einen Auslass für ein das Turbinenrad 8 antreibendes Fluid.

Das Außenzahnrad 17 des Untersetzungsgetriebes 10 bildet an seiner dem axial nachfolgenden Deflecting 2 zugewandten Stirnseite die eine Abtriebswelle 11, welche das innere Rohr 6 umgibt. Die Abtriebswelle 11 ist dabei zu einer Art Rohrstumpf ausgeformt, auf dessen radialer Außenseite das Deflecting 2 bspw. durch Kleben oder durch Vulkanisieren befestigt ist.

Dieses Deflecting 2 besteht im wesentlichen aus einer vorzugsweise zylinderförmigen Außenwand 2a, welche quasi als Fortsetzung der zylindrischen Außenwand 5 des Endoskopschafts 1 bzw. des Mittenabschnitts 12a des Rotationselements 4 vorgesehen ist. Radial innerhalb dieser Außenwand 2a, d.h. in dem Ringspalt zwischen dem auch das Deflecting 2 durchlaufenden Innenrohr 6 und der Außenwand 2a des Deflectings 2 befindet sich ein vorzugsweise im Querschnitt teilkreisförmiger Faltenbalg 20, der so in dem Deflecting 2 fixiert ist, dass er sich zusammen mit der Abtriebswelle des Untersetzungsgetriebes um die Längsachse des Endoskopschafts 1 auf einer Kreisbahn bewegt.

Gemäß der anliegenden Fig. 1 ist in dem dort dargestellten Innenrohr 6 eine sich radial erstreckende Öffnung 21 vorgesehen, über die der Faltenbalg 20 mit einem Druckmedium beaufschlagbar ist. Konkreter ausgedrückt befindet sich innerhalb oder am Innenrohr 6 eine Druckleitung (nicht weiter dargestellt), die in eine Ringkammer 22 in radialer Richtung mündet, welche innerhalb des die Getriebeabtriebswelle bildenden Rohrstumpfs 11 ausgeformt ist. Bei einer Rotation der Getriebeabtriebswelle 11 dreht sich somit die Ringkammer 22 mit, wobei jedoch eine permanente Fluidverbindung mit der Druckleitung infolge deren radiale Mündungsöffnung 21 verbleibt. Die Ringkammer 22 steht wiederum in Fluidverbindung mit dem Faltenbalg 20.

Bei einer Druckbeaufschlagung dehnt sich der Faltenbalg 20 in Folge seines konstruktiven Aufbaus vorzugsweise in Axialrichtung des Endoskopschafts 1 aus, wodurch aufgrund der einseitigen (exzentrischen) Anordnung des Faltenbalgs. 20 bezüglich der Mittelachse des Endoskopschafts 1 das Deflecting 2 eine Abkrümmbewegung in einer Ebene ausführt. Sobald das Druckmedium aus dem Faltenbalg 20 ausgelassen wird, zieht sich dieser in Längsrichtung gesehen wieder zusammen, wodurch das Deflecting seine ursprüngliche Lage wieder einnimmt oder sich noch weiter in die entgegengesetzte Richtung abkrümmt.

In der Fig. 4 ist der Endoskopkopf 3 des erfindungsgemäßen Endoskops schematisch in einem Seitenriß dargestellt.

Dieser besteht u.a. aus einem inneren rohrförmigen Stator 23, der dichtend mit dem inneren Rohr 6 verbunden ist. Auf dem Stator 23 ist ein Wälzlager 24 aufgesetzt, welches drehbar eine Kappe 25 am Stator 23 lagert, die eine Art becherförmiges Gehäuse des Endoskopkops 3 bildet. Im Innern der Kappe 25 sind eine Mehrzahl von Endoskopspezifischer Einrichtungen wie Beleuchtung, Optik, Sprühanlage und dergleichen an dieser fixiert, welche in der Fig. 3 nur schematisch dargestellt sind. Der Stator 23 ist an der Stirnseite der Kappe 25 offen, sodass medizinische Instrumente durch diesen aus dem Endoskop geschoben werden können.

Die Funktionsweise des vorstehend beschriebenen erfindungsgemäßen Endoskops gemäß dem bevorzugten Ausführungsbeispiel wird nachfolgend beschrieben:

Die in der Fig. 2 schematisch dargestellten Einlass- und Auslasskanäle 19 für den Antrieb der Turbine 8 sind vorzugsweise am proximalen Ende des Endoskopschafts 1 mit einer Druckquelle (nicht dargestellt) verbunden. Gleiches gilt auch für den nicht weiter gezeigten Druckfluidkanal innerhalb des oder am Innenrohr 6 zur Betätigung des Faltenbalgs 20, der ebenfalls mit einer separaten Druckquelle zur Betätigung des Deflectings 2 verbunden ist. Beide Druckquellen sind in der anliegenden Fig. 1 oder 2 zwar nicht dargestellt, können jedoch aus einer manuell betätigten Handpumpe oder einer Elektropumpe bestehen.

Wie eingangs kurz beschrieben wurde, ist es vorteilhaft, beim Einführgang des Endoskops in einen kanalartigen Hohlraum, bspw. den Darm eines Patienten, den distalen Endbereich des Endoskops entsprechend den zu durchlaufenden Windungen des Darms dynamisch anzupassen, indem das Deflecting 2 entsprechende Abkrümmbewegungen erzwungener Maßen ausführt. Hierzu wird wahlweise Fluid in den Faltenbalg 20 des Deflecting 2 gepresst, wodurch eine entsprechende Abkrümmbewegung des Deflecting 2 bewirkt wird. Da jedoch vorzugsweise nur ein einziger Faltenbalg 20 bezüglich der Mittellinie des Endoskopschafts 1 dezentral angeordnet ist, ist eine Abkrümmbewegung in nur einer Ebene (zweidimensional) möglich.

Um dieses auszugleichen, ist das erfindungsgemäße Rotationselement 4 zusätzlich vorgesehen.

Ist nämlich eine Abkrümmung des Deflecting 2 in unterschiedliche Richtungen (dreidimensional) erforderlich, wird über den Einlasskanal 19 ein Antriebsfluid in die Aufnahmekammer 13 der Turbine 7 unter einer vorbestimmten Strömungsgeschwindigkeit eingeleitet, wodurch das Turbinenrad 8 mit einer entsprechenden Umdrehungsgeschwindigkeit angetrieben wird. Diese Drehbewegung wird über den, einen Rotor darstellenden Rohrschaft 9 und das nachgeschaltete Untersetzungsgetriebe 10 mit einer entsprechenden Untersetzung auf die Abtriebswelle 11 des Untersetzungsgetriebes 10 übertragen, mit welchem das Deflecting 2 und damit auch der Faltenbalg 20 fixiert ist. Auf diese Weise wird das Deflecting 2 um dessen Längsachse gedreht.

Aufgrund der Tatsache, dass das Innenrohr 6 des Endoskopschafts 1, welches über den Endoskopschaft 1 hinaus verlängert ist und somit das Rotationselement 4 und das axial nachfolgende Deflecting 2 durchläuft, bezüglich des Rotationselements 4 sowie des Deflecting 2 rotatorisch entkoppelt und damit drehfest verbleibt, können auf diese Weise sämtliche darin enthaltenen Arbeits- und Versorgungskanäle sowie Leitungen, wie sie in der Fig. 3 andeutungsweise dargestellt sind, ohne Rotationsausgleichselemente verlegt sein.

Abschließend sei darauf hingewiesen, dass die vorstehend beschriebene Ausführungsform in unterschiedlicher Weise weiter verändert werden kann, ohne dass das eingangs beschriebene erfindungsgemäße Konzept verändert wird. So ist es denkbar, durch Verstellmöglichkeiten am Turbinenrad 8 die Rotationsgeschwindigkeit und ggf. sogar die Rotationsrichtung zu verändern.

Der Faltenbalg 20 wurde vorstehend als teilkreisförmig im Querschnitt beschrieben. Er könnte auch als ein normal gestalteter zylinderförmiger Faltenbalg ausgeführt sein.

## Patentansprüche

1. Endoskop mit einem Endoskopkopf (3), der über ein abwinkelbares Endstück (2) mit einem Endoskopschaft (1) zu einem rohrförmigen Bauteil verbunden ist, wobei zwischen dem Endoskopschaft (1) und dem abwinkelbaren Endstück (2) ein Rotationselement (4) für ein Rotieren des abwinkelbaren Endstücks (2) um die Längsachse des Endoskopschafts (1) eingefügt ist, **dadurch gekennzeichet, dass** das Rotationselement (4) einen Turbinenantrieb (7) hat bestehend aus einem Turbinenrad (8), das auf einem Rotor (9) sitzt und einem Stator (12a), welcher vorzugsweise eine Aufnahmekammer (13) für das Turbinenrad (8) ausbildet.

2. Endoskopkopf nach Anspruch 1, **dadurch gekennzeichnet, dass** das Rotationselement (4) einen Koaxialantrieb aufweist.

3. Endoskopkopf nach einem der vorstehenden Ansprüche, **dadurch gekennzeichnet, dass** das Rotationselement (4) ein Untersetzungsgetriebe (10) aufweist.

4. Endoskopkopf nach einem der vorstehenden Ansprüche, **dadurch gekennzeichnet, dass** das abwinkelbare Endstück (2) einen Abwinkelmechanismus aufweist, der ein Abwinkeln des Endstücks (2) nur in eine eingeschränkte Anzahl von Richtungen, vorzugsweise zweidimensional ermöglicht.

5. Endoskopkopf nach Anspruch 1, **dadurch gekennzeichnet, dass** der Rotor (9) mit einem Untersetzungsgetriebe (10) wirkverbunden ist, welches die Rotationsbewegung des Turbinenrads (8) mit einem bestimmten Untersetzungsverhältnis auf das abwinkelbare Endstück (2) überträgt.

6. Endoskopkopf nach Anspruch 5, **dadurch gekennzeichnet, dass** das Untersetzungsgetrieben (10) ein selbsthemmendes Getriebe der Axialbauweise und vorzugsweise ein Planetengetriebe oder ein Harmonie Drive ist.

7. Endoskopkopf nach einem der Ansprüche 5 oder 6, **dadurch gekennzeichnet, dass** der Stator (12a) eine Außenwand des Rotationselements (4) bildet, welche das Turbinenrad (8), den Rotor (9) sowie das nachgeschaltete Untersetzungsgetriebe (10) umgibt und vorzugsweise eine axiale Verlängerung des Endoskopschafts (1) darstellt.

8. Endoskopkopf nach Anspruch 7, **dadurch gekennzeichnet, dass** die Außenwand (12a) des Rotationselements (4) eine Führung und/oder Zentrierung für das Untersetzungsgetriebe (10) und insbesondere ein Hohlrad (18) des als Harmoniedrive oder Planetenradsatz ausgeführten Untersetzungsgetriebes (10) bildet.

9. Endoskopkopf nach einem der vorstehenden Ansprüche 5 bis 8, **gekennzeichnet durch** einen Einlass- und Auslasskanal (19), welche im Endoskopkopf (1) verlaufen und den Turbinenantrieb (7) mit einem Druckmedium beaufschlagen.

## Claims

1. Endoscope having an endoscope head (3) which is connected via a deflectable endpiece (2) to an endoscope shaft (1) to form a tubular component, a rotational element (4) being inserted between the endoscope shaft (1) and the deflectable endpiece (2) for rotation of the deflectable endpiece (2) about the longitudinal axis of the endoscope shaft (1), **characterised in that** the rotational element (4) has a turbine drive (7) comprising a turbine wheel (8) which sits on a rotor (9) and a stator (12a) which preferably forms a receiving chamber (13) for the turbine wheel (8).

2. Endoscope head according to claim 1, **characterised in that** the rotational element (4) has a coaxial drive.

3. Endoscope head according to one of the preceding claims, **characterised in that** the rotational element (4) has a reducing gear (10).

4. Endoscope head according to one of the preceding claims, **characterised in that** the deflectable endpiece (2) has a deflection mechanism which makes possible deflection of the endpiece (2) only in a restricted number of directions, preferably two-dimensionally.

5. Endoscope head according to claim 1, **characterised in that** the rotor (9) is operationally connected to a reducing gear (10) which transmits the rotational movement of the turbine wheel (8) with a specific gear reduction ratio to the deflectable endpiece (2).

6. Endoscope head according to claim 5, **characterised in that** the reduction gear (10) is a self-limiting gear of the axial construction and preferably a planetary gear or a harmonic drive.

7. Endoscope head according to one of the claims 5 or 6, **characterised in that** the stator (12a) forms an external wall of the rotational element (4), which wall surrounds the turbine wheel (8), the rotor (9) and also the subsequently connected reducing gear (10) and preferably represents an axial extension of the endoscope shaft (1).

8. Endoscope head according to claim 7, **characterised in that** the external wall (12a) of the rotational element (4) forms a guide and/or centring for the reducing gear (10) and in particular a hollow wheel (18) of the reducing gear (10) which is configured as a harmonic drive or planetary gear set.

9. Endoscope head according to one of the preceding claims 5 to 8, **characterised by** an inlet and outlet channel (19) which extend in the endoscope head (1) and supply the turbine drive (7) with a pressure medium.

## Revendications

1. Endoscope avec une tête d'endoscope (3), reliée, par l'intermédiaire d'une pièce d'extrémité (2) susceptible d'être coudée, à une tige d'endoscope (1) pour former un composant de forme tubulaire, entre la tige d'endoscope (1) et la pièce d'extrémité (2) susceptible d'être coudée étant inséré un élément de rotation (4), pour produire une rotation de la pièce d'extrémité (2) susceptible d'être coudée autour de l'axe longitudinal de la tige d'endoscope (1), **caractérisé en ce que** l'élément de rotation (4) comprend un entraînement à turbine (7), composé d'une roue de turbine (8), siégeant sur un rotor (9), et d'un stator (12a), constituant de préférence une chambre de logement (13) pour la roue de turbine (8).

2. Tête d'endoscope selon la revendication 1, **caractérisée en ce que** l'élément de rotation (4) présente un entraînement coaxial.

3. Tête d'endoscope selon l'une des revendications précédentes, **caractérisée en ce que** l'élément de rotation (4) présente une transmission cinématiquement réductrice (10).

4. Tête d'endoscope selon l'une des revendications précédentes, **caractérisée en ce que** la pièce d'extrémité (2) susceptible d'être coudée présente un mécanisme de déviation angulaire, permettant une déviation angulaire de la pièce d'extrémité (2), uniquement dans un nombre restreint de directions, de préférence de façon bidimensionnelle.

5. Tête d'endoscope selon la revendication 1, **caractérisée en ce que** le rotor (9) est relié fonctionnellement à une transmission cinématiquement réductrice (10), transmettant à la pièce d'extrémité (2) susceptible d'être coudée le mouvement de rotation de la roue de turbine (8), avec un rapport de réduction cinématique déterminé.

6. Tête d'endoscope selon la revendication 5, **caractérisée en ce que** la transmission cinématiquement réductrice (10) est une transmission auto-bloquante à mode de réalisation construction axial et, de préférence, une transmission planétaire ou une Harmonic Drive.

7. Tête d'endoscope selon l'une des revendications 5 ou 6, **caractérisée en ce que** le stator (12a) forme une paroi extérieure de l'élément de rotation (4), entourant la roue de turbine (8), le rotor (9), ainsi que la transmission cinématiquement réductrice (10) branchée en aval et, de préférence, constitue un prolongement axial de la tige d'endoscope (1).

8. Tête d'endoscope selon la revendication 7, **caractérisée en ce que** la paroi extérieure (12a) de l'élément élément de rotation (4) forme un guidage et/ou un centrage pour la transmission cinématiquement réductrice (10) et, en particulier, une roue creuse (18) de la transmission cinématiquement réductrice (10) réalisée sous forme d'Harmonic Drive ou de train de roue planétaire.

9. Tête d'endoscope selon l'une des revendications 5 à 8, **caractérisée par** un canal d'admission et d'échappement (19), s'étendant dans la tête d'endoscope (1) et sollicitant l'entraînement à turbine (7) avec un fluide sous pression.
